(19) **Europäisches Patentamt**
**European Patent Office**
**Office européen des brevets**

(11) Publication number: **0 183 527 B1**

(12) # EUROPEAN PATENT SPECIFICATION

(45) Date of publication of patent specification: **13.03.91**

(51) Int. Cl.⁵: **A61K 37/30, A61K 47/00**

(21) Application number: **85308583.5**

(22) Date of filing: **26.11.85**

Divisional application 90200083.5 filed on 26/11/85.

(54) Absorbable calcitonin medicament.

(30) Priority: **26.11.84 JP 248898/84**

(43) Date of publication of application:
**04.06.86 Bulletin 86/23**

(45) Publication of the grant of the patent:
**13.03.91 Bulletin 91/11**

(84) Designated Contracting States:
**DE FR GB**

(56) References cited:
EP-A- 0 115 627    EP-A- 0 122 036
EP-A- 0 156 772    FR-A- 2 312 260
FR-A- 2 313 914    GB-A- 2 127 689
GB-A- 2 142 335    US-A- 4 151 276

(73) Proprietor: **YAMANOUCHI PHARMACEUTICAL CO. LTD.**
**No. 3-11 Nihonbashi-Honcho, 2-chome Chuo-ku**
**Tokyo(JP)**

(72) Inventor: **Kagatani, Seiya**
**1506-6-304 Sangamyo**
**Yaizu shi Shizuoka(JP)**
Inventor: **Hasumi, Shunji**
**No. 6-2, Surugadai 2-chome**
**Fujieda-shi Shizuoka(JP)**
Inventor: **Sonobe, Takashi**
**13-8, Minamisurugadai 5-chome**
**Fujueda-shi Shizuoka(JP)**
Inventor: **Aruga, Masayoshi**
**1-7, Hinode-cho**
**Shizuoka-shi Shizuoka(JP)**

(74) Representative: **Geering, Keith Edwin et al**
**REDDIE & GROSE 16 Theobalds Road**
**London WC1X 8PL(GB)**

## Description

The present invention relates to intranasal [i.e. for administration via the nasal route] medical compositions containing calcitonin as an active ingredient together with absorption enhancer.

Calcitonin is a polypeptide hormone having various medical activities used for treatment of osteoporosis, hypercalcemia and Paget's disease.

Calcitonin, like other pharmacologically active peptides, is decomposed in the gastrointestinal tract by digestive juice, so that oral administration is ineffective. Due to this, and to its poor absorption, calcitonin is generally administered by injection, but this can be so painful that self-injection is unsuitable. Accordingly administration of calcitonin has been inconvenient and costly.

Recently, it has been found that nasal application of calcitonin gives effects similar to conventional muscular injection, and various intranasal calcitonin compositions have been proposed. However, a polypeptide of such large molecular weight as calcitonin has poor inherent nasal absorbability and therefore absorption enhancers, for example surface active agents, are generally incorporated [GB-A-2127689 and EP-A-115627]. In these proposals, both amphoteric and cationic surface active agents are employed, but it is said that nonionic surface active agents, inter alia polyoxyethylene lauryl ether, are particularly excellent as absorption accelerators. However, such an ether type surface active agent promotes the drug absorption by destroying the nasal membrane. Thus, the preferred surface active agent has a strong toxicity to tissue and so is unsatisfactory for practical use alone.

From extensive investigations we have found effective enhancers for the nasal absorption of calcitonin which are suitable for practical use. The present invention provides an intranasal medical composition comprising [a] calcitonin and [b] at least one absorption enhancer therefor selected from thiamine and salts thereof, salicylic acid and salts thereof, capric acid and salts thereof, pyridoxal and salts thereof, malic acid and salts thereof and pyrophosphoric acid and salts thereof.

As calcitonin for the present invention, various kinds such as salmon calcitonin, human calcitonin, elcatonin and porcine calcitonin, can be used.

Among the absorption enhancers for the present invention, compounds capable of forming salts may be used in free and/or salt form. Suitable salts of basic compounds [such as pyridoxal and thiamine, are the hydrochloride and nitrate; suitable salts of acidic substances [such as pyrophosphoric acid, salicylic acid, malic acid and capric acid] are the sodium and potassium salts.

The absorption enhancers may be used singly or in combinations of two or more.

The intranasal medical composition of the present invention may be in the form of an aqueous solution, hydrogel or solid powder.

An aqueous solution can be prepared by dissolving calcitonin and absorption enhancer in water or a buffer solution in conventional manner. In this case, additive is added to and dissolved in the aqueous solution, if necessary. It is preferred that pH of the aqueous solution be between 3 and 5, for stability.

As the buffer, citrates, tartarate and malates can be employed, in a preferred pH range of 3 to 5.

As additive, one or more substances selected from sterilizers, preservatives, tackifiers, surface active agents and stabilisers conventionally used for intranasal agents can be incorporated.

Conventional sterilisers and preservatives may be used and examples include p-oxybenzoates, propylene glycol, benzetonium chloride and sorbic acid [Na].

As tackifiers, polyvinyl alcohol, polyvinyl pyrrolidone and dextran may be employed.

Surface active agent can be added as dispersing and emulsifying agent for various additives; nonionic surface active agents that cause little irritation to the nasal mucous membrane are preferred. As these nonionic surface active agents, for example, polyoxyethylene monostearate, polyoxyethylene sorbitan monooleate and polyoxyethylene-hydrogenated castor oil are used.

As stabiliser, mention may be made of gelatin or albumin.

The solution can be administered dropwise or by spraying using a dropping container, a sprayer or a nasal aerosol applicator.

For a powder composition, mannitol, inositol or glucose can be added in conventional manner; after dissolving and then freeze-drying, the resulting solid is pulverised into fine powder to be administered by the nasal route. Such powder may for example be packed in a capsule, the capsule set in a spraying device and penetrated by a needle to make pores at its top and bottom, and air applied [e.g. via a rubber bulb] to blow the powder out. When volatile liquid components are used as the absorption enhancers, the powdery form is not suitable.

In the case of an aqueous gel, calcitonin can be formed into the aqueous gel using conventional gel bases, for example, natural gums, methyl celluloses, acrylic polymers, vinyl polymers and polysaccharides.

The proportions of calcitonin as active ingredient, absorption enhancer and various additives to be used in the medical composition of the present invention are not particularly limited but are appropriately determined depending upon dosage form [solution, gel or powder]. In the case of an aqueous solution for intranasal drops, calcitonin is suitably formulated in a concentration of from 200 to 600 IU/ml, preferably 500 to 2000 IU/ml; the individual dose is preferably 0.05 to 0.2 ml and administered preferably 1 to 5 times daily. The amount of absorption enhancer incorporated varies, but in the case of an aqueous solution it is appropriately from 0.05 to 15% [w/v]; a particularly preferred range is 1.0 to 10% [w/v] for ethanol and 0.1 to 5% [w/v] for other absorption enhancers.

By the use of a specific absorption enhancer in the calcitonin intranasal agent of this invention, efficiency of absorbing across the membrane of the nasal cavity is improved.

The present invention is illustrated in more detail by the following Examples but is not to be limited thereto.

### Preparation Example 1

|  | per 1 ml |
|---|---|
| Salmon calcitonin | 350 IU |
| Citric acid hydrate | 12.2 mg |
| Sodium citrate | 12.4 mg |
| Absorption enhancer | [cf. Table 1] |

### TABLE 1

| | Absorption Enhancer | Amount [mg] |
|---|---|---|
| a | Control | — |
| e | Malic acid | 30 |
| f | Sodium caprate | 10 |
| g | Sodium salicylate | 10 |
| i | Thiamine hydrochloride | 10 |
| j | Pyridoxal hydrochloride | 10 |
| k | Sodium pyrophosphate | 30 |
| l | Sodium pyrophosphate | 30 |
| | Thiamine hydrochloride | 30 |
| m | Sodium pyrophosphate | 30 |
| | Benzyl alcohol | 10 |

700 IU salmon calcitonin was dissolved in less than 1 ml of a solution containing 24.4 mg citric acid. Absorption enhancer in an amount twice that shown in Table 1 was dissolved in less than 1 ml of a solution containing 24.8 mg sodium citrate. After pH adjustment to 4.0 using 1N aqueous hydrochloric acid or 1N aqeuous sodium hydroxide, water was added to make 1 ml of each solution. The equal volumes were mixed to provide 2 ml of the preparation having the required concentration. The salicylate preparation was a suspension. The sodium caprate preparation was adjusted to pH 8.

Example 1

Sprague Dawley strain male rats[115-145 g] fasted for 18 hours were anaesthetized with pentobarbital [50 mg/kg, intraperitoneal injection]. Aqueous calcitonin preparations [a] to [m] as in Preparation Example 1 were administered to the rats in a dose of 5 IU/kg.

Administration was performed by using a microsyringe [2 μl] connected with a polyethylene tube [FE 10, Clay Adams] and injecting about 2 μl at a distance of 5 to 6 mm from the nasal septum depending upon the body weight. Evaluation of absorption of the calcitonin preparation through the nasal membrane was performed by measuring the concentration of calcium in serum. The calcium concentration was quantitatively determined using a calcium meter [CA-30, Joko]. Rats were sacrificed prior to administration and 1, 2 and 3 hours after administration. Blood was collected from the descending large vein. The results are showin in Table 2. The data shown in Table 2 are mean values of 3 or more rats.

## TABLE 2

Concentration of Calcium in Serum after Intranasal Administration of Calcitonin [5 IU/kg]

|   | Absorption Enhancer | Concen- tration % | Ca mg % 1h. | 2h. | 3h |
|---|---|---|---|---|---|
| a | Control | – | 10.65 | 10.24 | 10.81 |
| e | Malic acid | 3 | 8.87 | 11.59 | 10.24 |
| f | Sodium caprate | 1 | 8.67 | – | – |
| g | Sodium salicylate | 1 | 8.56 | 9.33 | 10.71 |
| i | Thiamine hydrochloride | 1 | 8.48 | 9.59 | 11.37 |

4

| j | Pyridoxal | | | | |
|---|---|---|---|---|---|
| | hydrochloride | 1 | 8.85 | – | 11.70 |
| k | Sodium | | | | |
| | pyrophosphate | 3 | 8.97 | 9.21 | 9.84 |
| l | Sodium pyrophosphate | 3 | | | |
| | Thiamine | | – | 8.68 | – |
| | hydrochloride | 3 | | | |
| m | Sodium pyrophosphate | 3 | | | |
| | Benzyl alcohol | 1 | – | 8.64 | – |

Calcium concentration in serum prior to administration: 10.67 mg%.

From Table 2, it is noted that Ca concentration in serum is significantly reduced by the addition of a specific absorption enhancer compared to the control.

In a manner similar to Preparation Example 1, calcitonin intranasal compositions shown in the following Preparation Examples were obtained. These calcitonin solutions provide reduction of the calcium concentration in serum as in Example 1.

### Preparation Example 2

| | |
|---|---|
| Procine calcitonin | 1400 IU |
| Benzyl alcohol | 10 mg |
| Malic acid | 13.4 mg |
| Methyl p-oxybenzoate | 5 mg |
| Propyl p-oxybenzoate | 2 mg |

1N aqueous sodium hydroxide was added to adjust pH to 4.0. Water was added to make the final volume 1 ml.

### Preparation Example 3

| | |
|---|---|
| Elcatonin | 1400 IU |
| Salicylic acid | 10 mg |
| Benzetonium chloride | 0.1 mg |
| Tartaric acid | 30 mg |
| Hydroxypropyl cellulose | 20 mg |

1N aqueous sodium hydroxide was added to adjust pH to 4.0. Water was added to make the final volume 1 ml.

Preparation Example 4

| | |
|---|---|
| Salmon calcitonin | 1400 IU |
| Macrogol 400 | 10 mg |
| Thiamine hydrochloride | 10 mg |
| Citric acid monohydrate | 12.2 mg |
| Sodium citrate | 12.4 mg |

Water was added to make the final volume 1 ml.

Preparation Example 5

| | |
|---|---|
| Salmon calcitonin | 14000 IU |
| Sodium pyrophosphate | 300 mg |
| D-Ribose | 300 mg |
| Benzetonium chloride | 1 mg |
| Citrate acid monohydrate | 122 mg |
| Sodium citrate | 124 mg |

5N Hydrochloride acid was added to adjust pH to 4.0. Water was added to make the final volume 10 ml.

Claims

1. An intranasal medicament composition comprising [a] calcitonin and [b] at least one absorption enhancer selected from thiamine and salts thereof, salicylic acid and salts thereof, capric acid and salts thereof, pyridoxal and salts thereof, malic acid and salts thereof and pyrophosphoric acid and salts thereof.

2. A container containing a composition as claimed in claim 1 and provided with means enabling application of the contained composition to the nasal mucosa.

3. A container according to claim 2 provided with means enabling application of the contained composition to the nasal mucosa in spray form.

4. An applicator device containing a composition as claimed in claim 1 and provided with means enabling application of the contained composition to the nasal mucosa in spray form.

Revendications

1. Une composition de médicament intranasal comprenant (a) de la calcitonine et (b) au moins un améliorateur d'absorption choisi parmi l'alcool benzoïque, la thiamine et ses sels, l'acide salicylique et ses sels, l'acide caprique et ses sels, le pyridoxal et ses sels, l'acide malique et ses sels, et l'acide pyrophosphorique et ses sels.

2. Un récipient contenant une composition telle que revendiquée à la revendication 1 et muni de moyens permettant l'application de la composition contenue à la muqueuse nasale.

3. Un récipient selon la revendication 2, muni de moyens permettant l'application de la composition contenue à la muqueuse nasale sous forme de pulvérisation.

4. Un dispositif applicateur contenant une composition telle que revendiquée à la revendication 1 et muni de moyens permettant l'application de la composition contenue à la muqueuse nasale sous forme de pulvérisation.

## Ansprüche

1. Intranasale Arzneimittelzubereitung, umfassend (a) Calcitonin und (b) mindestens ein die Absorption verstärkendes Mittel, ausgewählt aus Thiamin und Salzen davon, Salicylsäure und Salzen davon, Caprinsäure und Salzen davon, Pyridoxal und Salzen davon, Maleinsäure und Salzen davon und Pyrophosphorsäure und Salzen davon.

2. Behälter, enthaltend eine Zubereitung nach Anspruch 1 und versehen mit Mitteln, die das Aufbringen der darin enthaltenen Zubereitung auf die Nasenschleimhaut ermöglichen.

3. Behälter nach Anspruch 2, versehen mit Mitteln, die das Aufbringen der darin enthaltenen Zubereitung auf die Nasenschleimhaut in Form eines Sprays ermöglichen.

4. Applikatorvorrichtung, enthaltend eine Zubereitung nach Anspruch 1 und versehen mit Mitteln, die das Aufbringen der darin enthaltenen Zubereitung auf die Nasenschleimhaut in Form eines Sprays ermöglichen.